# EUROPEAN PATENT SPECIFICATION

(11) **EP 2 231 040 B1**
(45) Date of publication and mention of the grant of the patent: **09.04.2014**
(21) Application number: 07870457.4
(22) Date of filing: 12.12.2007
(51) Int. Cl.: A61B 17/42

(54) **SINGLE USE, DISPOSABLE, UTERINE MANIPULATOR**
EINWEG-GEBÄRMUTTERMANIPULATOR ZUR EINMALIGEN VERWENDUNG
MANIPULATEUR UTERIN A USAGE UNIQUE JETABLE

(43) Date of publication of application: 29.09.2010
(73) Proprietor: Vectec, 03270 Hauterive (FR)
(72) Inventor: D'ARPIANY, Francis, F-03110 Vendat (FR); ARBOY, Yves, FR-92200 Neuilly sur Seine (FR)
(74) Representative: Thurgood, Alexander John
(86) International application number: PCT/IB2007/004428
(87) International publication number: WO 2009/074844

(56) References cited:
- WO-A-96/11641
- FR-A- 2 745 171
- US-A- 1 245 845
- US-A- 2 400 251
- US-A- 2 822 809
- US-A- 3 766 907
- US-A- 3 777 743
- US-A- 5 746 750

## Description

The present invention relates to single use, disposable surgical instruments in general, and more particularly to a device for manipulating a uterus that is particularly adapted to use in coelioscopic surgery.

One of the current problems encountered in coelioscopic surgery in female patients is the position that the female reproductive organs, and in particular the uterus, the fallopian tubes and ovaries, adopt because of the way the patient is laid out on the operating table. This position, know as the Trendelebourg position, causes the female reproductive organs to fall back into the Douglas' pouch or rectouterine excavation, thereby making access to that pouch difficult or impossible for the surgeon. Several attempts have been made to produce devices that would assist the surgeon in gaining access to the Douglas' pouch more easily, most of which involve inflicting some kind of trauma on the female reproductive organs.

For example, one known device comprises a frustoconical screw-threaded plug that is screwed into the cervix of the female patient, in order to permit manipulation of the uterus, and thereby enable the surgeon to position the uterus as desired during surgery. The problem with a device such as this is that the screw thread bites into the cervical wall, and inevitably causes a certain degree of trauma. It has been noticed in general that trauma to the cervical wall, in whatever form, can lead to reduced fertility, especially in younger female subjects, and so it would be desirable to avoid, where possible, inflicting any such trauma.

Another example of a device for uterine manipulation involves an inflatable balloon, that is provided at a distal tip of a manipulator. The manipulator is inserted into the uterus via the vagina, and the balloon inflated so that the exterior walls of the balloon come into pressure contact with the inner walls of the uterus. The idea is that the expansion of the balloon causes sufficient friction against the inner uterine wall to enable the surgeon to operate the manipulator and thereby displace the uterus in the desired direction. However, in order to be effective, the device must generally be inserted into the uterus until it meets with the upper inner wall of the latter, and then the balloon inflated, and the pressure thereby exerted by the balloon can cause localised damage to the uterine inner wall. Document US 2,822,809 discloses do uterine manipulator according to the preamble of independent claim 1.

The device according to the present invention attempts to overcome the above limitations, whilst at the same time being easy to use and operate, and most notably, being atraumatic, i.e. not inflicting trauma on the inner wall of the uterus or the cervical wall.

Accordingly, one object of the present invention is a single use, disposable uterine manipulator device, comprising :
- a tubular elongate portion defining a longitudinal axis of the device, and having a proximal and a distal extremity ;
- wherein the distal extremity is provided with sealing abutment means for sealingly abutting the cervix, and the distal extremity is further provided with uterine wall suction means distributed around the distal extremity for providing suction to the device, thereby enabling said device to come into suction contact with an inner uterine wall;
wherein the sealing abutment means and the uterine wall suction means are formed integrally as a sleeve that fits over the distal extremity of the tubular elongate portion and
wherein the sleeve can preferably have a section that widens from a proximal end towards a distal end of said sleeve.

Preferably, the tubular elongate portion is made of a sterilisable material, preferably stainless steel, however other materials may also provide satisfaction, provided that they are suitable for use in a surgical instrument, are suitably rigid and can be sterilised.

Preferably, the sleeve of the uterine wall suction means is made of a sterilisable polymer material, and even more preferably of a polymer material selected from the group consisting of polyoxymethacrylate, polyacetal, and PA66.

In accordance with a preferred embodiment, the tubular elongate portion is open at the distal extremity and together with an inner surface of the sleeve mentioned above defines a space allowing for the passage of fluid. The passage of fluid can be simply formed by providing for a difference in diameter between the outside diameter of the tubular elongate portion and the inner diameter of the sleeve, and leaving the distal extremity of the tubular elongate portion free or unblocked, such that fluids may pass either into the distal extremity of the elongate portion, for example, air being withdrawn under vacuum, or in the opposite direction, for example, when a liquid, such as a contrast or washing agent, is injected from the proximal end of the tubular elongate portion.

The sleeve also preferably comprises a plurality of suction orifices which traverse a wall of the sleeve and communicate with the space allowing for passage of fluid. Thes plurality of orifices is even more preferably distributed about the sleeve in a manner enabling a sufficient portion of the sleeve to come into suction contact with an inner uterine wall. Thus the plurality of orifices forms, along with the space created between the sleeve and elongate tubular portion, a way of creating fluid communication between an inner wall of the uterus, and the outside environment, i.e. the environment outside of the uterus.

Where fluid communication to be created is in the form of vacuum or suction, such suction is preferably provided at a vacuum pressure comprised between 0.4 bar and 0.8 bar, and most preferably is about 0.6 bar. The applicant has noticed that this latter value is completely atraumatic for the tissue of the uterus, including the inner walls of the uterus to which the device becomes attached when vacuum or suction is applied through the device.

Even more preferably, the device sleeve comprises a total of 8, or most preferably 10 orifices, distributed evenly about the sleeve in a 3-2-3, or respectively, a 3-2-3-2 configuration. Such a configuration allows for optimal coverage of the inner uterine wall surfaces, thereby facilitating manipulation of the uterus when vacuum is being applied.

The sealing abutment means are designed to abut, in a sealing manner, the cervix, and thus it is preferred that they be located at a proximal end of the sleeve, and have a section that is substantially conical, adapted in shape and size to sealingly abut a cervix, and extending from a widened proximal end to a narrowed distal end which abuts or adjoins the proximal end of the sleeve. It is to be noted that since the sealing abutment means are substantially conical, the nose or distal end formed thereby can preferably be snubbed or rounded, to enable a less traumatic contact with the cervix. One should note here the complete contrast with prior art solutions involving a screw thread which engages the tissue of the cervix and can cause damage thereto.

In one advantageous embodiment, the device further preferably comprises grip means, mounted in axial sliding engagement around the tubular elongate portion, proximal to the uterine wall suction means and sealing abutment means, and distal to the proximal extremity of the tubular elongate portion. Preferably, the grip means are mounted in fixed rotating engagement about the tubular elongate portion, but they can also be made to rotate in and out of a locked position, should that be desired. It can thus be seen that the tubular elongate portion bearing the uterine wall suction means can slide along inside the grip means, while leaving an exterior grip surface free for the surgeon to manipulate the device, for example, rotating it in one direction or another.

Even more preferably, the device also comprises a Luer lock 2-way valve located at the proximal extremity the tubular elongate portion. Such a 2-way Luer lock preferable provides :
- in a first open position, for a fluid communication between the elongate tubular portion and the uterine wall suction means,
- in a second position, for complete obstruction of the Luer lock.

In still yet another preferred embodiment, a surgical kit comprising the above mentioned uterine manipulator device in combination with an hysterectomy guide means. These guide means are used to aid the surgeon in performing a hysterectomy, either a sub-total hysterectomy or a total hysterectomy, depending on how much of the cervix is removed along with the uterus. Preferably, the hysterectomy guide means comprises an adapter comprising engagement means to engage the proximal end of the sealing abutment means, and ablation guide sleeve which is adapted to engage at least partially circumferentially an exterior wall of the cervix. Even more preferably, the adapter has clips means for removably engaging a proximal surface of the sealing abutment means. It can thus be seen that the adapter slides over the sealing abutment means and can be clipped and retained at a proximal end of the sealing abutment means. Where the adapter is made of resilient or slightly elastic material, it is preferably designed to elastically fit over the substantially conical sealing abutment means and then snap back over a proximal circumference or periphery of said sealing abutment means.

As a result, it is preferred that the adapter also be substantially conical in shape, extending from a widened proximal end towards a narrowed distal end, the shape defined thereby enabling sealing abutment of the adapter with an inner vaginal wall. Thus, the initial cervical sealing abutment means no longer serve their purpose, since they are covered, in this particular embodiment, by the adapter, which is dimensioned to provide sealing abutment with an inner vaginal wall and not the cervix.

In a further preferred embodiment, the ablation guide sleeve is substantially tubular, in order to be able to be slid over the device as described initially, but also comprises a peripheral projecting wall, which extends from a proximal end in abutment with the distal end of the adapter, towards a distal end, wherein the projecting wall engages at partially circumferentially an exterior wall of the cervix. In this way, the projecting wall serves as a guide for the surgeon as to where the tissue of the cervix should be sectioned and removed along with the rest of the uterus.

Other objects and preferred embodiments will become apparent through the detailed description and the figures, given purely for illustrative purposes, as follows :

### Brief Description of the Figures

Figure 1 is schematic represenation of a single use, disposable, uterine manipulation device according to the present invention ;
Figure 2a, 2b and 2c are different schematic views of a distal part of the uterine manipulation device illustrated in Figure 1 ;
Figure 3 is a first representation of the introduction into a uterus of the device of Figure 1 ;
Figure 4 is a second representation of the normal final position of the device of Figure 1 once fully introduced into the uterus ;
Figures 5a, 5b and 5c are different schematic representations of a further adapter for the device of the present invention ;
Figure 6 is a schematic representation of an extra component that fits onto the adapter illustrated in Figure 5, and which is suited to assisting a surgeon in carrying out a hysterectomy.

### Examples, or Detailed Description of the Invention

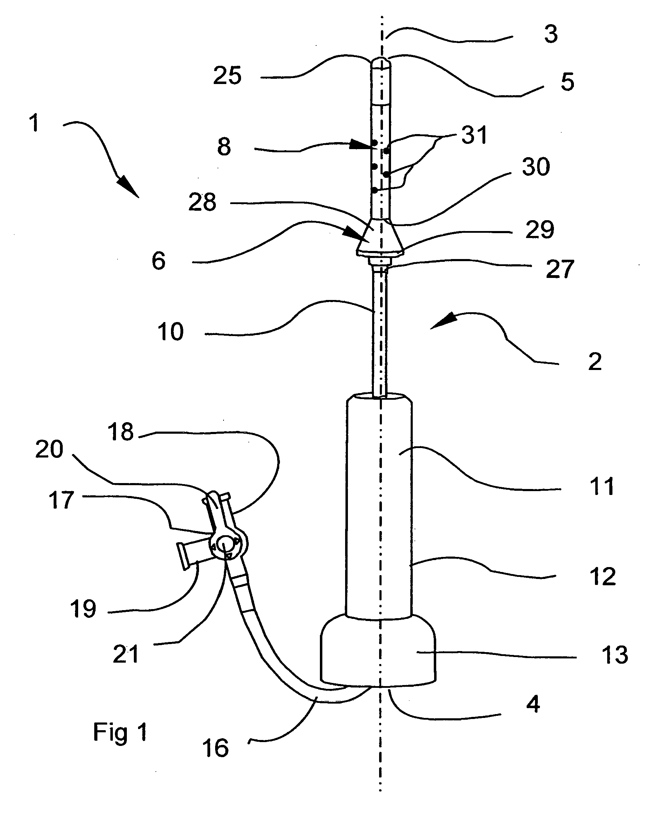

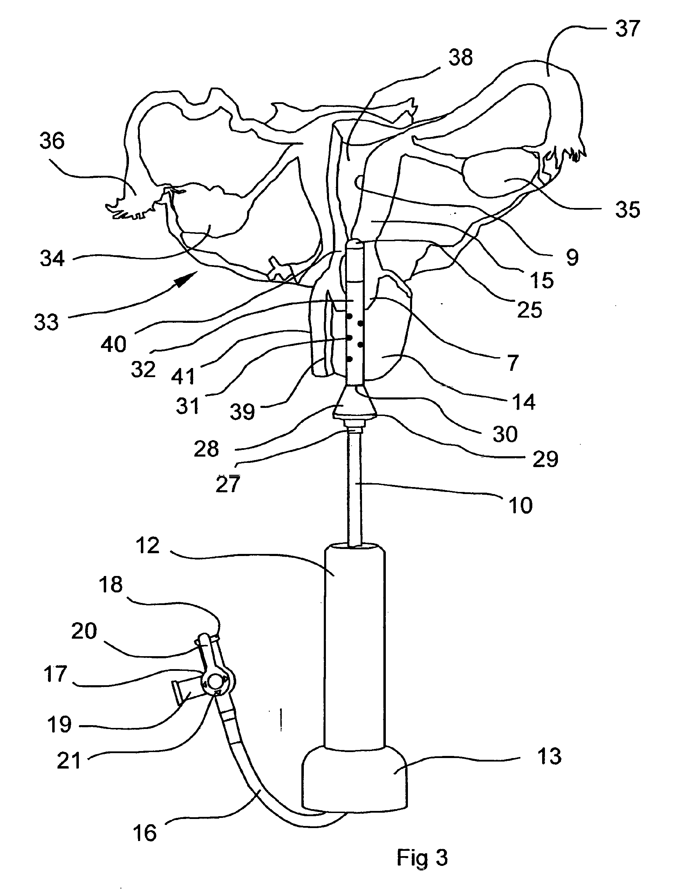

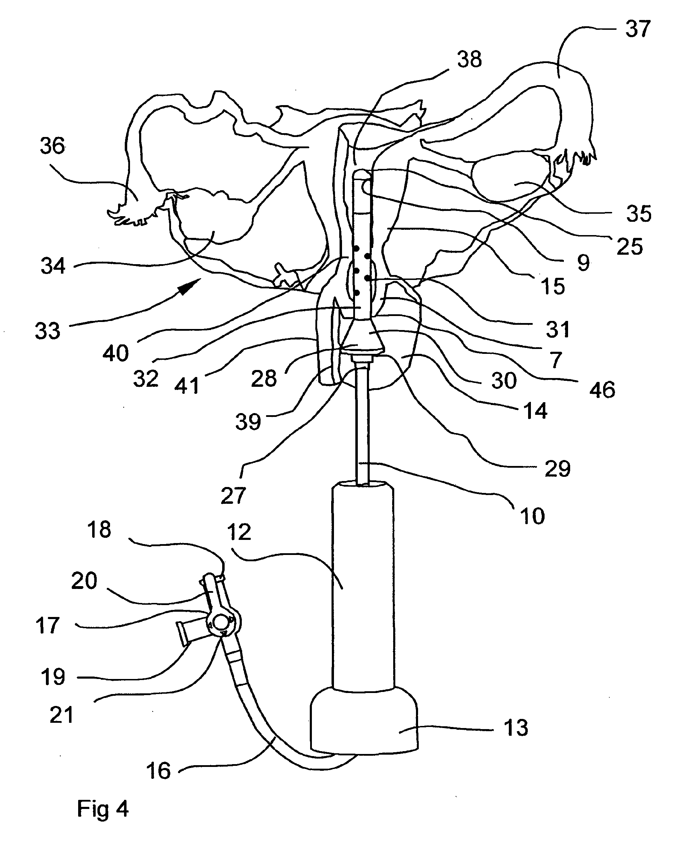

As can be seen from Figure 1 , the single use, disposable, uterine manipulation device, indicated by the general reference I, comprises a tubular elongate portion, identified by the general reference 2, defining a longitudinal axis 3 of the device I, and having a proximal 4 and a distal extremity 5. The distal extremity 5 is provided with sealing abutment means, indicated generally by the reference 6, for sealingly abutting a cervix 7 (cf. Figures 3 and 4), and the distal extremity 5 is further provided with uterine wall suction means 8 distributed around the distal extremity 5 for providing suction to the device I, thereby enabling the device I to come into suction contact with an inner uterine wall 9. The tubular elongate portion 2 is made of a sterilisable, surgically appropriate material, preferably stainless steel, and thus generally takes the shape of a stainless steel tube 10. The tube 10 is essentially hollow along the axis 3 and has an opening at both the proximal 4 and distal 5 extremities. Figure 1 also shows that the device I further comprises grip means, indicated generally by the reference 11, mounted in axial sliding engagement around the tubular elongate portion 2, proximal to the uterine wall suction means 8 and sealing abutment means 6 and distal to the proximal extremity 4 of the tubular elongate portion 2. The grip means 11 are mounted in fixed rotating engagement about the tubular elongate portion 2. As can be seen from Figure 1 , the grip means 11 comprise a fairly large outer diameter tube 12. The tube can optionally be provided with ridges, not illustrated in the figure, to facilitate prehension by the operator of the device 1. The grip means 11 further include an enlarged, approximately funnel-shaped, proximal extremity 13, enabling other instruments to be introduced into the tube 12 if necessary. Since the grip means 11 are rotatively, but notslidingly locked, about the axis 3 of the device, it is posssible to manipulate the device by rotating the tube 12 about said axis. It is furthermore possible to slide the sealing abutment means 6 and uterine wall suction means 8 along the axis 3 to introduce the latter into, and out of, the vagina 14 towards the uterus 15. The proximal extremity 4 of the device I is connected to a fluid communication conduit 16. The conduit 16 is made of a flexible fluid impermeable polymer material, of the type commonly used in hospitals or other surgical devices for the passage of fluids, be they liquids or gases. The fluid communication conduit 16 is connected to a Luer lock valve 17, having two ports 18, 19. A first port 18 is connected to a vacuum generator device or apparatus (not shown), for establishing vacuum or suction, such that air is sucked in via the uterine wall suction means 8, through the distal extremity 5 of the device I, and then along the tubular elongate portion 2 and through the conduit 16 and first port 18. A second port 19 is provided for introduction and removal of another fluid, for example, a contrast agent such as methylene blue, which can be injected as a liquid through the second port 19 and into the uterus 15 via the device I by following the same schema as above, but in a reverse direction. The 2-way Luer lock valve 17 thus provides, in a first open position, for a fluid communication between the elongate tubular portion 2 and the uterine wall suction means 8, and in a second position, for complete obstruction of the Luer lock 17. The 2 Luer lock valve 17 thus also comprises a switch or lever 20, which can be activated to set the lock in a desired position, depending on whether the operator of the device I wants the fluid communication to be vacuum or suction in the first case, fluid communication for the introduction of a contrast agent, in a second case, and of course a third position, whereby all fluid communication is obstructed. In order to facilitate recognition of the position in which the lock 17 is set, the lock can be provided with markings 21, arranged on the lever 20, and indexed therewith.

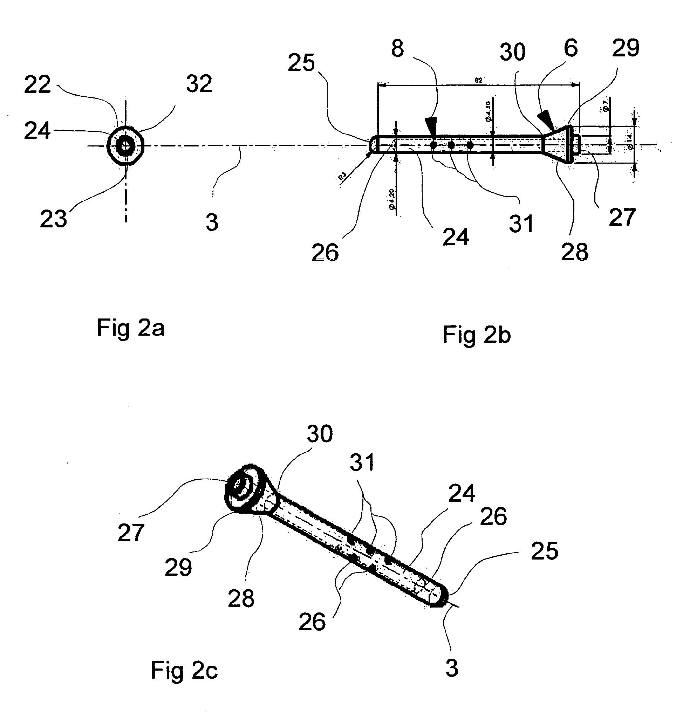

As can be seen in more detail from Figure 2, the sealing abutment means 6 and the uterine wall suction means 8 are formed integrally as a sleeve 32 that fits over the distal extremity 5 of the tubular elongate portion 2. Figure 2a shows a cross-section of the sleeve 32, along axis 3 of the device, clearly showing that the sleeve is hollow, and has an outer wall 22 and inner wall 23, defining an inner bore or space 24. The distal end 25 of the sleeve is blind, i.e. closed, and the distal limit 26 of the bore or space 24 has a generally concave shape, such that a distal end of the stainless steel tube 10 does not sealingly abut said limit 26, thereby allowing for passage of fluid between an opening in the distal end of the tube 10, and the space 24 of the sleeve. Additionally, as can be seen from Figure 2b, the sleeve does not have a circular cross-section, but extends from proximal end 27 towards the distal end 25 of the sleeve in a slightly widening cross-section, thereby ensuring that a fluid communication space 24 will be created between the stainless steel tube 10 and the inner wall 23 of the sleeve 32, and that the tube 10 will be held in elastic sealing engagement with the proximal end 27. The sealing abutment means 6 are located at a proximal end 27 of the sleeve 32, and has a cross-section susbtantially forming a cone 28, adapted in shape and size to sealingly abut a cervix. The cone 28 extends from a widened proximal end 29 to a narrowed distal end 30 which abuts or adjoins the sleeve 32. The uterine wall suction means 8 also comprise a plurality of orifices 31, distributed evenly around the sleeve 32, preferably in a 3-2-3-2 configuration or arrangement, as can be seen partially from Figure 2c. The orifices 31 traverse the outer and inner walls 22, 23 of the sleeve, and open into the fluid communication space 24. Thus, when vacuum is applied via the Luer lock port 18, air is withdrawn from within the device and a depression created that causes a uterine wall 9 to be sucked onto the sleeve 32. In a converse manner, when fluid, such as a contrast agent, is introduced via port 19, said fluid can pass through the device, into the space 24 and through the orifices into the uterus 15. As the cone sealingly abuts the cervix 7 of the uterus 15, no leakage in the sealtight environment can occur, and thus the vacuum pressure is maintained, or respectively, the fluid introduced into the uterus 15 remains therein or will permeate through the tissues therein or other organs connected thereto.

Turning now to Figure 3, one can see a schematic representation of female human reproductive organs, indicated by the general reference 33. These organs are represented generally by the ovaries 34, 35, the fallopian tubes 36, 37, the uterine cavity 38, the cervix 7, the uterine isthmus 40, and the vagina 14. The vagina 14 and uterus are shown here o the right hand side a in partial cut-away representation, in order to show the uterine cavity 38 and inner 39 and outer vaginal 41 walls and inner uterine 9 walls. In Figure 3, one can see that the device has been introduced via the vagina 14 and the cervix into the uterus 38, such that the distal end 25 of the sleeve 32 has reached the uterine isthmus 40. In this position, the device I of the invention is not yet ready for use.

Figure 4 shows, in a schematic representation, the position of the device I of the invention, once introduction thereof into the uterus 15 has been completed. In this figure, it can be seen that the distal end 30 of the cone 28 of the sealing abutment means has come into sealing abutment engagement with the cervix 7, forming a substantially leaktight seal. One will also note that the sleeve 32 has now been pushed past the uterine isthmus 40, such that the distal end 25 of the sleeve 32 is located in the uterine cavity 38. The plurality of orifices 31 of the suction means 8 are now in physical contact with an inner uterine wall 9.In Figure 4, the Luer lock port 18 is shown in the closed position, but when the lever 20 is moved and set to the open position, i.e. in this particular example by turning the lever through 90 degrees so that it lies over the second port 19, then vacuum pressure that can be applied will suck out any air in the device, creating a depression in the space 24 between the inner wall of the sleeve 32 and stainless steel tube and thereby causing a drop in pressure at the orifices 31, which in turn will cause the inner uterine wall 9 to be sucked onto the outer wall 22 of the sleeve 32. It thus becomes possible to manipulate the uterus 15 and associated organs and put them into a position more suitable for the surgeon, simply by moving the device around in the three-dimensional anatomical space. In this way, it also makes the Douglas' pouch accessible to the surgeon, should that be required. The seal is maintained by the conical shape of the cone 28 which abuts sealingly against the cervix 7, independently of the angle of inclination of the device. In a similar maner, a diagnostic test, for example, the methylene blue test, can be carried out very simply by activating the lever 20 again, to set the Luer lock port 18 into the closed position, for example, by either moving the lever 20 forward again by 90 degrees, or by reversing the initial movement. This movement will then open port 19, through which the methylen blue contrast agent can be injected. As there is a leaktight seal against the cervix 7, none of the fluid introduced can escape and will therefore diffuse in a classical manner through the various tissues, and particularly up the fallopian tubes to the ovaries, where the contrast agent will become visible through a coelioscope.

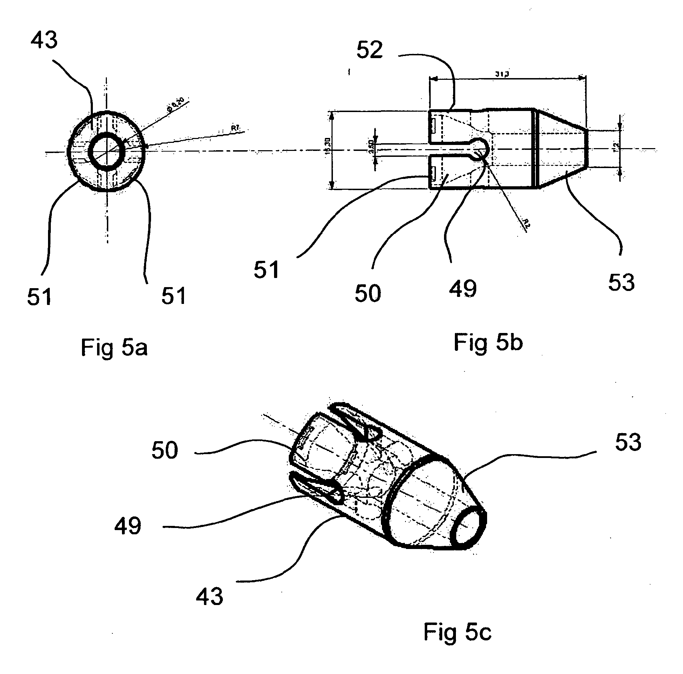

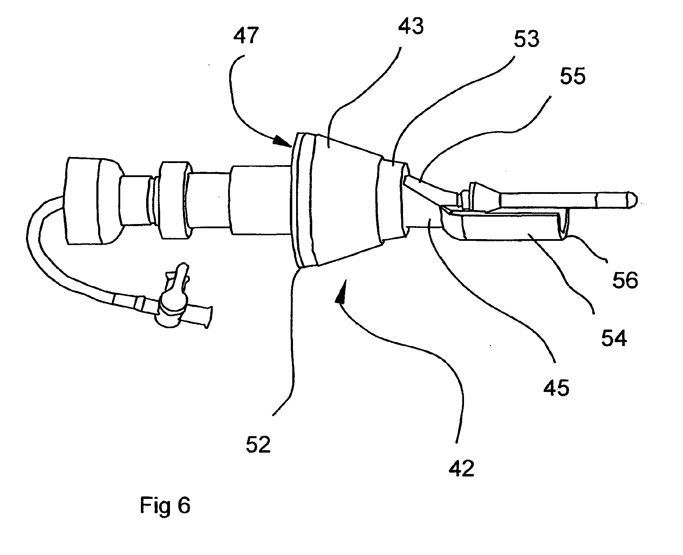

Turning now to Figures 5 and 6, these are schematic representations of a further embodiment of the device of the present invention, in which said uterine manipulator device I can be adapted to guide the surgeon who needs to carry out a hysterectomy, be it a total or sub-total hysterectomy. Consequently, the device I further comprises hysterectomy guide means, indicated by the general reference 42 comprises an adapter 43 comprising engagement means 44 to engage the proximal end 27 of the sealing abutment means 6, and ablation guide sleeve 45 which is adapted to engage at least partially circumferentially an exterior wall 46 of the cervix 7. The adapter 43 preferably has clip means 47 for removably engaging a proximal surface 48 of the sealing abutment means 6. In Figure 5, it can be seen that the adapter 43 has lands or cut-outs 49, which form wings 50 in the adapter 43 and enable it to be slid over the cone 28 via the distal end of the sealing abutment means 6. Since the adapter is preferably made of resilient or elastic material, the wings 50 spread as the adapter 43 is slid over the cone 28 towards the widened proximal end 29 of the latter. The clip means 47, which can for example be one or more radial flanges 51 provided on the wings 50, then reach over the widened proximal end 29 of the cone and clip into place on a contact surface 48 which is essentially the proximal surface of the widened proximal end 29. Such a clipping action provides for sufficient maintainance of the adapter 43 in position, whilst at the same time allowing for its release simply by easing a radial flange back over onto the cone 28. Consequently, the adapter 43 is preferably substantially conical in shape, extending from a widened proximal end 52 towards a narrowed distal end 53, the shape defined thereby enabling sealing abutment of the adapter 43 with an inner vaginal wall 39. The ablation guide sleeve 45 also comprises a peripheral projecting wall 54, which extends from a proximal end 55 in abutment with the distal end 53 of the adapter 43, towards a distal end 56, wherein the projecting wall 54 engages partially circumferentially an exterior wall of the cervix 46. In this way, the projecting wall 54 serves to guide the surgeon wishing to remove the uterus 15 down to a limit on the cervix 7 that is defined by the extent of the projecting peripheral wall 54.

## Claims

1. Single use, disposable, uterine manipulator device (1) comprising :
- a tubular elongate portion (2) defining a longitudinal axis (3) of the device, and having a proximal (4) and a distal (5) extremity ;
- wherein the distal extremity (5) is provided with sealing abutment means (6) for sealingly abutting a cervix, and the distal extremity (5) is further provided with uterine wall suction means (8) distributed around the distal extremity (5) for providing suction to the device, thereby enabling said device (1) to come into suction contact with an inner uterine wall; and
- wherein the sealing abutment means (6) and the uterine wall suction means (8) are formed integrally as a sleeve (32) that fits over the distal extremity (5) of the tubular elongate portion (2), **characterised in that**
the sleeve (32) has a cross-section that widens from a proximal end (27) towards a distal (25) end of said sleeve.

2. Uterine manipulator device according to claim 1, wherein the tubular elongate portion (2) is made of a sterilisable material, preferably stainless steel.

3. Uterine manipulator device according to claim 1, wherein the sleeve (32) is made of a sterilisable polymer material, preferably selected from the group consisting of polyoxymethacrylate, polyacetal, and PA66.

4. Uterine manipulator device according to any of claims 1 to 3, wherein the tubular elongate portions (2) is open at the distal extremity (5) and together with an inner surface (23) of the sleeve (32) defines a space allowing for the passage of fluid.

5. Uterine manipulator device according to any of the preceding claims, wherein the sleeve (32) comprises a plurality of suction orifices (31) which traverse a wall of the sleeve (32) and communicate with the space allowing for passage of fluid.

6. Uterine manipulator device according to claim 5, wherein the plurality of orifices (31) is distributed about the sleeve (32) in a manner enabling a sufficient portion of the sleeve (32) to come into suction contact with an inner uterine wall.

7. Uterine manipulator device according to any of the preceding claims, wherein the suction is provided at a vacuum pressure comprised between 0.4 bar and 0.8 bar, and preferably is about 0.6 bar.

8. Uterine manipulator device according to any of the preceding claims, wherein the sleeve (32) comprises a total of 8 orifices (31), distributed evenly about the sleeve in a 3-2-3 configuration, or even more preferably 10 orifices (31), distributed evenly about the sleeve (32) in a 3-2-3-2 configuration.

9. Uterine manipulator device according to any of the preceding claims, wherein the sealing abutment means (6) are located at a proximal end of the sleeve (32), and has a section that is substantially conical, adapted in shape and size to sealingly abut a cervix, and extending from a widened proximal end (29) to a narrowed distal end (30) which abuts or adjoins the sleeve (32)

10. Uterine manipulator device according to any of the preceding claims, wherein the device further comprises grip means (11), mounted in axial sliding engagement around the tubular elongate portion (2), proximal to the uterine wall suction means (8) and sealing abutment means (6) and distal to the proximal extremity (4) of the tubular elongate portion (2).

11. Uterine manipulator device according to any of the preceding claims, wherein the grip means (11) are mounted in fixed rotating engagement about the tubular elongate portion (21).

12. Uterine manipulator device according to any of the preceding claims, wherein the device further comprises a Luer lock 2-way valve (17) located at the proximal extremity (4) of the tubular elongate portion (2).

13. Uterine manipulator device according to claim 12, wherein the 2-way Luer lock (17) provides :
- in a first open position, for a fluid communication between the elongate tubular (2) portion and the uterine wall suction means (8),
- in a second position, for complete obstruction of the Luer lock.

14. Surgical kit comprising a uterine manipulator device according to any of the preceding claims, and a hysterectomy guide means (42).

15. Surgical kit according to preceding claim 14, wherein the hysterectomy guide means (42) comprises an adapter (43) comprising engagement means (44) to engage the proximal end (27) of the sealing abutment means (6), and ablation guide sleeve (45) which is adapted to engage at least partially circumferentially an exterior wall of the cervix.

16. Surgical kit according to preceding claim 15, wherein the adapter (43) has clips means (47) for removably engaging a proximal surface (48) of the sealing abutment means (6).

17. Surgical kit according to preceding claim 15, wherein the adapter (43) is substantially conical in shape, extending from a widened proximal end (52) towards a narrowed distal end (53), the shape defined thereby enabling sealing abutment of the adapter (43) with an inner vaginal wall.

18. Surgical kit according to preceding claims 17, wherein the ablation guide sleeve (45) comprises a peripheral projecting wall (54), which extends from a proximal end (55) in abutment with the distal end (53) of the adapter (43) towards a distal end (56), wherein the projecting wall (54) engages partially circumferentially an exterior wall of the cervix.

## Patentansprüche

1. Wegwerfbare, einweg Gebärmuttermanipulationsvorrichtung, umfassend:
- eine rohrförmige längliche Abschnitt (2), welche eine Längsachse (3) der Vorrichtung definiert, mit einem proximalen Ende (4) und einem distalen Ende (5);
- wobei das distale Ende (5) mit Dichtungsangrenzmitteln (6) zum abdichtenden Angrenzen an einen Gebärmutterhals vorgesehen ist, und das distale Ende (5) weiter mit Gebärmutterwandssaugmittel (8) vorgesehen ist, welche verteilt um das distale Ende (5) sind, um eine Saugaktion der Vorrichtung zu verleihen, wobei dadurch die Vorrichtung (1) in Saugkontakt mit einer Innenwand der Gebärmutter bemächtigt wird; und
- wobei die Dichtungsangrenzmittel (6) und die Gebärmutterwandssaugmittel integral als eine Hülse (32) geformt werden, welche über das distale Ende (5) des röhrenförmigen länglichen Abschnitts paßt, **dadurch gekennzeichnet, dass** die Hülse (32) mit einem Querschnitt vorgesehen ist, der sich von einem distalen Ende (27) zu einem distalen Ende (25) der Hülse aufweitet.

2. Gebärmuttermanipulationsvorrichtung gemäss Anspruch 1, wobei der rohrförmige längliche Abschnitt (2) aus sterilisierbarem Material, bevorzugt aus Edelstahl, besteht.

3. Gebärmuttermanipulationsvorrichtung gemäss Anspruch 1, wobei die Hülse (32) aus sterilisierbarem Polymermaterial besteht, und vorzugsweise aus der Gruppe bestehend aus polyoxymethacrylate, Polyacetal und PA66 ausgewählt wird.

4. Gebärmuttermanipulationsvorrichtung gemäss irgendeinem der Ansprüche 1 bis 3, wobei der rohrförmige längliche Abschnitt (2) am distalen Ende (5) offen ist, und wobei dieser, zusammen mit einer inneren Oberfläche (23) der Hülse (32), einen Raum definiert, welcher den Durchfluss einer Flüssigkeit ermöglicht.

5. Gebärmuttermanipulationsvorrichtung gemäss irgendeinem der vorhergehenden Ansprüche, wobei die Hülse (32) eine Mehrzahl von Saugöffnungen (31) umfasst, welche eine Wand der Hülse (32) durchquert und mit dem Raum kommuniziert, welcher den Durchfluss einer Flüssigkeit ermöglicht.

6. Gebärmuttermanipulationsvorrichtung gemäss Anspruch 5, wobei die Mehrzahl von Saugöffnungen (31) um die Hülse (32) so verteilt wird, dass es einem ausreichendem Teil der Hülse (32) ermöglicht, in Saugkontakt mit einer Innenwand der Gebärmutter zu kommen.

7. Gebärmuttermanipulationsvorrichtung gemäss irgendeinem der vorhergehenden Ansprüche, wobei das Saugen bei einem Vakuumdruck zwischen 0,4 bar und 0,8 bar, und vorzugsweise etwa 0,6 bar, bewirkt wird.

8. Gebärmuttermanipulationsvorrichtung gemäss irgendeinem der vorhergehenden Ansprüche, wobei die Hülse (32) 8 Öffnunngen (31) umfasst, welche gemäß einer 3-2-3 Konfiguration gleichmäßig um die Hülse verteilt sind, oder vorzugsweise 10 Öffnungen (31) umfasst, welche gemäß einer 3-2-3-2 Konfiguration gleichmäßig um die Hülse (32) verteilt sind.

9. Gebärmuttermanipulationsvorrichtung gemäss irgendeinem der vorhergehenden Ansprüche, wobei die Dichtungsangrenzmittel (6) an einem proximalen Ende der Hülse (32) angeordnet sind, und einen im Wesentlichen konischen Abschnitt aufweisen, welcher in Form und Grösse angepasst ist, um an einen Gebärmutterhals dichtend anzugrenzen, und welcher sich von einem breiteren proximalen Ende (29) zu einem distalen verengten Ende (29) erstreckt, und letzteres die Hülse anliegt oder an die Hülse grenzt.

10. Gebärmuttermanipulationsvorrichtung gemäss irgendeinem der vorhergehenden Ansprüche, wobei die Vorrichtung ferner Greifmittel (11) umfasst, die in axialen Gleiteingriff um den rohrförmigen länglichen Abschnitt (2) montiert sind, und proximal im Vergleich zu den Gebärmutterwandssaugmitteln (8) un den Dichtungsangrenzmitteln (6), und distal im Vergleich zum proximalen Ende (4) des rohrförmigen länglichen Abschnitts (2).

11. Gebärmuttermanipulationsvorrichtung gemäss irgendeinem der vorhergehenden Ansprüche, wobei die Greifmittel (11) so montiert sind, dass sie in fester Drehung um den röhrenförmigen länglichen Abschnitt (2) stehen.

12. Gebärmuttermanipulationsvorrichtung gemäss irgendeinem der vorhergehenden Ansprüche, wobei die Vorrichtung ferner ein am distalen Ende des rohrförmigen länglichen Abschnitts (2) vorgesehenes 2-Wege-Luer-Lock-Ventil (17) umfasst.

13. Gebärmuttermanipulationsvorrichtung gemäss Anspruch 12, wobei das 2-Wege-Luer-Lock-Ventil (17):
- in einer ersten offenen Position, den Durchfluss einer Flüssigkeit zwischen dem röhrenförmigen länglichen Abschnitt (2) und den Gebärmutterwandssaugmitteln (8);
- in einer zweiten Position, die totale Obstruktion des Luer-Locks
ermöglicht.

14. Chirurgisches Kit umfassend eine Gebärmuttermanipulationsvorrichtung nach einem der vorhergehenden Ansprüche, und Hysterektomie-Führungsmittel (42).

15. Chirurgisches Kit gemäss vorhergehendem Anspruch 14, wobei die Hysterektomie-Führungsmittel (42) einen Adapter (43) umfasst, der auch Eingriffsmittel (44) umfasst, welche das proximale Ende (27) der Dichtungsangrenzmittel (6) in Eingriff nehmen, und eine Ablationsführungshülse (45), die angepasst ist, mindestens teilweise umlaufend mit einer Aussenwand des Gebärmutterhals einzugreifen.

16. Chirurgisches Kit gemäss vorhergehendem Anspruch 15, wobei der Adapter (43) mit Klemmmittel (47) vorgesehen ist, um lösbar eine proximale Oberfläche (48) der Dichtungsangrenzmittel (6) einzugreifen.

17. Chirurgisches Kit gemäss vorhergehendem Anspruch 15, wobei der Adapter (43) im wesentlichen eine konische Form aufweist, und sich von von einem breiteren proximalen Ende (52) zu einem verengten distalen Ende (53) erstreckt, wobei die dadurch definierte Form ein dichtendes Angrenzen des Adapters (43) an einer innere Scheidenwand ermöglicht.

18. Chirurgisches Kit gemäss vorhergehendem Anspruch 17, wobei die Ablationsführungshülse (45) eine vorstehende Umfangswand (54) umfasst, welche sich von einem proximalen Ende (55), das an das distalen Ende (53) des Adapters (43) angrenzt, zu einem distalen Ende (56) erstreckt, und wobei die vorstehende Umfangswand (54) sich mindestens teilweise umlaufend mit einer Aussenwand des Gebärmutterhals eingreift.

## Revendications

1. Dispositif (1) manipulateur utérin jetable à usage unique, comprenant :
- une partie (2) tubulaire allongée définissant un axe (3) longitudinal du dispositif, et présentant une extrémité proximale (4) et une extrémité distale (5) ;
- dans lequel l'extrémité distale (5) est munie de moyens (6) d'étanchéité venant en butée pour venir en butée de manière étanche contre un col d'utérus et l'extrémité distale (5) est en outre pourvue de moyens d'aspiration (8) de la paroi utérine répartis autour de l'extrémité distale (5) pour fournir une aspiration au dispositif, permettant ainsi audit dispositif (1) d'entrer en contact aspirant avec une paroi intérieure de l'utérus; et
- dans lequel les moyens d'étanchéité (6) venant en butée et les moyens d'aspiration de la paroi utérine sont formés d'un seul tenant en tant que manchon (32) qui s'adapte sur l'extrémité distale (5) de la partie allongée tubulaire (2), **caractérisé en ce que** le manchon (32) présente une section transversale qui s'élargit depuis une extrémité proximale (27) vers une extrémité distale (25) dudit manchon.

2. Dispositif manipulateur utérin selon la revendication 1, dans lequel la partie tubulaire allongée (2) est constituée d'un matériau stérilisable, de préférence de l'acier inoxydable.

3. Dispositif manipulateur utérin selon la revendication 1, dans lequel le manchon (32) est constitué d'un matériau polymère stérilisable, de préférence choisi dans le groupe consistant en du polyoxyméthacrylate, du polyacétal, et du PA66.

4. Dispositif manipulateur utérin selon l'une quelconque des revendications 1 à 3, dans lequel la partie tubulaire allongée (2) est ouverte à l'extrémité distale (5) et forme, en coopération avec une surface intérieure (23) du manchon (32), un espace permettant le passage de fluide.

5. Dispositif manipulateur utérin selon l'une quelconque des revendications précédentes, dans lequel le manchon (32) comprend une pluralité d'orifices d'aspiration (31) qui traverse une paroi du manchon (32) et communique avec l'espace permettant le passage de fluide.

6. Dispositif manipulateur utérin selon la revendication 5, dans lequel la pluralité d'orifices (31) est répartie autour du manchon (32) dans une manière permettant à une partie suffisante du manchon de venir en contact aspirant une paroi utérine intérieure.

7. Dispositif manipulateur utérin selon l'une quelconque des revendications précédentes, dans lequel l'aspiration est fournie à une pression de vide comprise entre 0.4 bar et 0.8 bar, et de préférence est de l'ordre de 0.6 bar.

8. Dispositif manipulateur utérin selon l'une quelconque des revendications précédentes, dans lequel le manchon (32) comprend un total de 8 orifices (31), répartis de manière égale autour du manchon selon une configuration 3-2-3, ou de manière encore plus préférée 10 orifices (31), répartis de manière égale autour du manchon (32) selon une configuration 3-2-3-2.

9. Dispositif manipulateur utérin selon l'une quelconque des revendications précédentes, dans lequel les moyens d'étanchéité (6) venant en butée sont situés à une extrémité proximale du manchon (32), et présentent une section qui est sensiblement conique, adaptés en forme et en taille pour venir en butée étanche contre un col d'utérus, et s'étendant depuis une extrémité proximale élargie (29) vers une extrémité distale amincie (29) qui vient en butée, ou qui vient s'adjoindre, au manchon.

10. Dispositif manipulateur utérin selon l'une quelconque des revendications précédentes, dans lequel le dispositif comprend en outre des moyens de préhension (11), montés pour venir s'engager de manière axiale autour de la partie tubulaire allongée (2), et proximale par rapport aux moyens d'aspiration (8) de la paroi utérine et aux moyens d'étanchéité (6) venant en butée, et distale par rapport à l'extrémité proximale (4) de la partie tubulaire allongée (2).

11. Dispositif manipulateur utérin selon l'une quelconque des revendications précédentes, dans lequel les moyens de préhension (11) sont montés de manière à s'engager en rotation fixe autour de la partie tubulaire allongée (2).

12. Dispositif manipulateur utérin selon l'une quelconque des revendications précédentes, dans lequel le dispositif comprend en outre une valve à deux sens avec raccord Luer (17) située à l'extrémité proximale de la partie tubulaire allongée.

13. Dispositif manipulateur utérin selon la revendication 12, dans lequel la valve à deux sens avec raccord Luer (17) permet :
- dans une première position ouverte, le passage de fluide entre la partie allongée tubulaire (2) et les moyens d'aspiration (8) de la paroi utérine,
- dans une deuxième position, l'obstruction complète du raccord Luer.

14. Nécessaire chirurgical comprenant un dispositif manipulateur utérin selon l'une quelconque des revendications précédentes, et des moyens (42) de guidage de l'ablation de l'utérus.

15. Nécessaire chirurgical selon la revendication 14 précédente, dans lequel les moyens (42) de guidage de l'ablation de l'utérus comprend un adaptateur (43) comprenant des moyens d'engagement (44) pour engager l'extrémité proximale (27) des moyens d'étanchéité (6) venant en butée, et un manchon de guidage (45) de l'ablation qui est adapté à s'engager au moins partiellement de manière circonférentielle avec une paroi extérieure du col de l'utérus.

16. Nécessaire chirurgical selon la revendication 15 précédente, dans lequel l'adaptateur (43) présente des moyens formant clips (47) pour engager de manière amovible une surface proximale (48) des moyens d'étanchéité (6) venant en butée.

17. Nécessaire chirurgical selon la revendication 15 précédente, dans lequel l'adaptateur (43) présente une forme sensiblement conique, s'étendant depuis une extrémité proximale élargie (52) vers une extrémité distale amincie (53), la forme ainsi définie permettant la venue en butée de manière étanche de l'adaptateur (43) avec une paroi vaginale intérieure.

18. Nécessaire chirurgical selon la revendication 17 précédente, dans lequel le manchon de guidage d'ablation (45) comprend une paroi périphérique en saillie (54), et qui s'étend depuis une extrémité proximale (55) en butée avec l'extrémité distale (53) de l'adaptateur (43), vers une extrémité distale (56), dans lequel la paroi en saillie (54) s'engage de manière partielle et circonférentielle avec une paroi extérieure du col de l'utérus.
